# EUROPEAN PATENT APPLICATION

(11) **EP 0 821 978 A2**
(43) Date of publication of application: **04.02.1998**
(21) Application number: 97305745.8
(22) Date of filing: 30.07.1997
(51) Int. Cl.: A61M 16/00, A61M 25/02

(54) **Light weight positive air pressure breathing apparatus**

(30) Priority: 30.07.1996 US 688438
(71) Applicant: Hart-Ellereth Medical Inc., Palatine, Illinois 60067 (US)
(72) Inventor: Ellereth, David, Illinois 60014 (US)
(74) Representative: Cardwell, Stuart Martin

(57) **Abstract**

Apparatus for generating positive pressure breathing air for patients suffering from obstructive sleep apnea including a nasal assembly comprising plural tube segments each fitted with a nostril engagement member to engage the interior walls and lower edge tissue of the nostril thereby providing both an air-tight seal as well as retention of the apparatus to the patient's nose. No other head retention gear or straps are required. A stress-relieving assembly is provided to interconnect one or more tubes from the nasal assembly to the source of pressurized air. A family of nostril engagement members may be provided to achieve a proper nostril fit. The engagement members may be angled to conform with ordinary nostril divergence.

## Description

The present invention relates to sleep apnea and, more specifically, to Continuous Positive Airway Pressure (CPAP) apparatus used during sleep to treat the sleep disorder known as obstructive sleep apnea (OSA).

Sleep apnea is a disorder characterized by pauses in, or the cessation of, the normal breathing function. Several forms of sleep apnea have been identified including central sleep apnea, a neurological condition in which respiratory activity actually ceases during the episode, and obstructive sleep apnea in which respiration efforts continue, but where the air passage becomes blocked thereby inhibiting normal breathing. As noted, the present invention pertains to this latter form of apnea.

As its name implies, this disorder occurs during sleep and often results from the relaxation of the muscles in the upper air passage (throat) which, in turn, causes a restriction or total collapse of that passage. If untreated, sleep apnea may have serious health consequences. Although rarely fatal (the reduction in blood oxygen associated with apnea usually triggers patient 'awakening'), the repeated interruption of 'restful' sleep causes daytime drowsiness and may lead to heart problems and other health disorders.

The principal medical treatment for sleep apnea is known as nasal CPAP *(i.e.* Continuous Positive Air Pressure). CPAP is not a cure. Rather, CPAP addresses the underlying pathology by maintaining an artificially elevated air pressure which literally inflates - - thereby assuring - - that such passage remains open.

It requires little imagination to appreciate that apparatii suitable for pressuring a user's breathing passageway are cumbersome and, themselves, not conducive to restful sleep. Masks, for example, require harnesses comprised of headgear, myriad straps, and clips - - all-but requiring the wearer to sleep on his/her back. Air leaks-- not uncommon between the mask and user's face - - create noise, compromise system performance, as well as generating annoying air 'blasts', *e.g.* an air stream directed toward the wearer's eyes. Tightening the mask, in an attempt to minimize such leaks, creates undue mask-to-face pressure which, in turn, frequently results in painful facial pressure points or, worse, actual skin bruises and sores.

These problems are not the sole province of mask-based CPAP solutions. Indeed, other known systems are similarly encumbered. For example, the so-called "nose pillow" - - characterized by its parallel pair of conically-tipped tubular members - - must be urged into abutting engagement with the user's nostrils, again, through the use of headgear, straps and clips. See also U.S. patent to Landis, No. 5,477,852, in which a pair of inflatable tubular members is inserted into the user's nostrils but, again, positioned and retained by headband and supporting straps.

The present invention seeks to avoid the above-noted physical limitations of masks and straps through the adoption of a novel nose cannula arrangement in which sufficient cannula sealing and nare engagement are achieved at the distal cannula ends whereby the associated air delivery tubing is arranged and retained in proper orientation without reliance on other headgear retaining apparatus.

More specifically, the respective ends of the air-supply tubes of the present CPAP delivery system are held in a spaced-apart, converging relationship by an injection molded plastic nasal assembly. This assembly is, itself, comprised of two rigid tube segments maintained in relative fixed relationship by a bridge member. The first ends of each tube segment have a flexible air supply hose connected thereto and second and distal ends thereof include nostril engagement members which, in turn, are adapted, as set forth below, for insertion into the user's nostrils. The lateral dimension between such distal ends approximates typical nostril separation while the converging or angled relationship of the tube segments facilitates the interconnection of the flexible air-delivery tubes as well as approximating the angular flare commonly defined by the human nose. The specific spacing and angle between nose bridge tube segments may be of fixed dimension or it may be adjustable to accommodate a wider variety of users. The nasal assembly may be integrally fabricated or assembled from separate components.

A family of soft nasal interface O-ring like members or seals are provided and define the respective ends of the nostril engagement members. Any matched set of engagement members may be positioned on the tube segments as appropriate for the user. Preferably the O-ring seals are selected to be snugly received within the nostrils whereby the slightly oversized cross-sections thereof (*i.e.* oversized in relation to the user's nostrils) are frictionally retained therein. This frictional fit functions additionally as an air seal thereby eliminating the previously noted annoyance of air leaks. Retention of the interface member may be further augmented through a clamping action in which the ends of the air-supply tubes are biased together, in turn, providing a clamping pressure that grips the nasal septum Alternatively, the engagement member O-rings may have a cross-section substantially identical to the corresponding cross-section of the user's nostrils. In such case, retention and sealing is augmented by an abutting contact that naturally occurs between the O-ring and the lower 'lip' portion of the nostril, i.e. the enlarged tissue region defining the visible nostril inlet. Finally, further support for the nasal assembly may be provided by the air supply hoses which, while intrinsically flexible, nevertheless can be utilized to provide assembly support

In the preferred arrangement a clip and strap is provided to stress relieve the flexible air-delivery tube(s) to permit the user to 'roll-about' in bed without dislodging the actual nasal interface. However, by reason of the intrinsic nasal locking character of the present design, no straps, caps, or other headgear is required Instead, it is contemplated that the strap will be positioned around the users chest (much like a convention trouser belt, although somewhat higher on the torso) and, as such, will permit the full rotation and movement of the head and neck. All that remains to interconnect the patient's nose is one or two flexible tube/hoses.

It is therefore an object of the present invention to provide an improved CPAP delivery system that will enable the patient to realize the benefits of positive air passage pressure during sleep for treating or avoiding episodes of obstructive sleep apnea while obviating the often negating effects associated with conventional CPAP air delivery systems. It is an object that the nasal connection provide a substantially air-tight interface with the patient's nose whereby air does not leak or 'blow-by' such interface thereby avoiding annoying 'air-blasts' against the user's eyes and face In turn, it is a further object that the interface, with its comparatively tight air seal to the nose, not generate significant noise which, itself, could serve to interfere with the sound sleep of the user. It is yet a further object that the required retention and air seal be achieved without the use of straps, clips, caps and other cumbersome headgear which restrict a patient's free movement and, too, interfere with restful sleep. It is an object that positive pressure air be delivered to both nostrils through one or more delivery tubes that terminate either within the nasal passages, themselves, or are attached to an interface member which, in turn, engages said nasal passages. It is an object that such engagement be achieved with an adjustable cross-section device or alternatively with a family of nasal interface O-rings, or the like, whereby the user may select an appropriately dimensioned nostril engagement device as required to achieve the above-described air-tight seal. The interface member may be adapted to receive a matched pair of engagement devices and, further, may be arranged to retain such engagement devices in fixed, but adjustable if desired, spaced-apart relationship and, yet further, angled such as to simulate the normal divergence of human nasal passages. It is a further object that a clamping, biasing action may further be imparted to the interface member and engagement devices to achieve, if desired, additional nose retention by reason of a clamping action to the septum. It is yet a further object that a stress-relief device be provided to retain the air supply delivery tube(s), but in such manner that the user may freely move his neck and head, said stress-relief device may preferably be a clip and strap adapted for positioning around the waste or upper torso of the user. These and other objects will be apparent from the following figures and detailed description of the preferred embodiments.

The present invention will now be described further hereinafter, by way of example only, with reference to the accompanying drawings;
Figure 1 is a perspective view of the CPAP air delivery system of the present invention shown in operation on a patient during sleep;
Figure 2 is a front elevation view of the nasal interface and stress relief portions of the present CPAP air delivery system with the nasal interface shown in use on and within a patients nasal passage;
Figure 3 is a front elevation view of a tube segment with nostril engagement member thereon of the nasal interface portion of Figure 2;
Figures 4a through 4e are front elevation views of five differently dimensioned nostril engagement members defining a family of such members;
Figure 5a is a front elevation view of the bridge member of the nasal interface portion of Figure 2;
Figure 5b is a top plan view of the bridge member of Figure 5a; and,
Figure 6 illustrates an alternative single air supply embodiment of the CPAP delivery system of Figure 1.

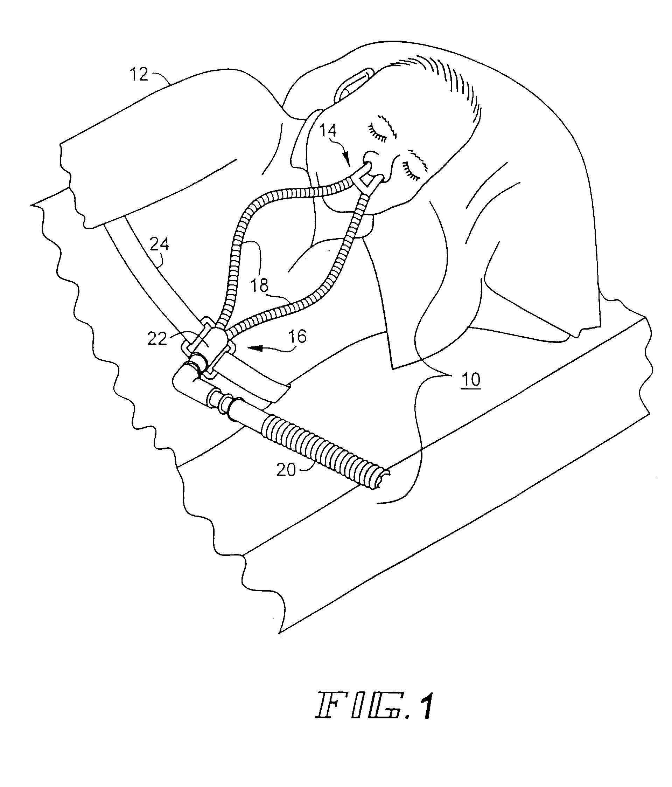

Figure 1 illustrates the CPAP air delivery system **10** of the present invention properly positioned on, and in use by, a patient **12** who suffers from OSA-type sleep apnea. What makes this real-life sketch unusual, at least in connection with sleep apnea patients fitted with a positive air pressure delivery system, is the fact that patient **12** is asleep on his side, head resting comfortably, sideways, on the bed pillow. Known alternative CPAP delivery systems are, at best, uncomfortable particularly unless the user lies on his back.

The complete OSA-type apnea CPAP system is comprised generally of a nasal interface assembly **14**, a waist /chest stress-relieving assembly **16**, and a pair of flexible, 3/8" intermediate air delivery hoses **18**. Also required, although forming no part of the present invention, is an air pump (not shown) to provide a source of pressured breathing air through a 3/4" hose **20**. A Y-adapter **22** is fixed mounted to, and forms a part of, the stress-relieving assembly **16**. Y-adapter **22** serves to split the single source of air from hose **20** between the twin-hoses **18** that supply the corresponding inlets of the nasal interface assembly. Hoses **18** are preferably in the order of 14" in length although longer or shorter lengths may be employed depending on the patient size and precise body placement. Stress-relieving assembly **16** is retained by a strap **24** of conventional design positioned around the user's torso.

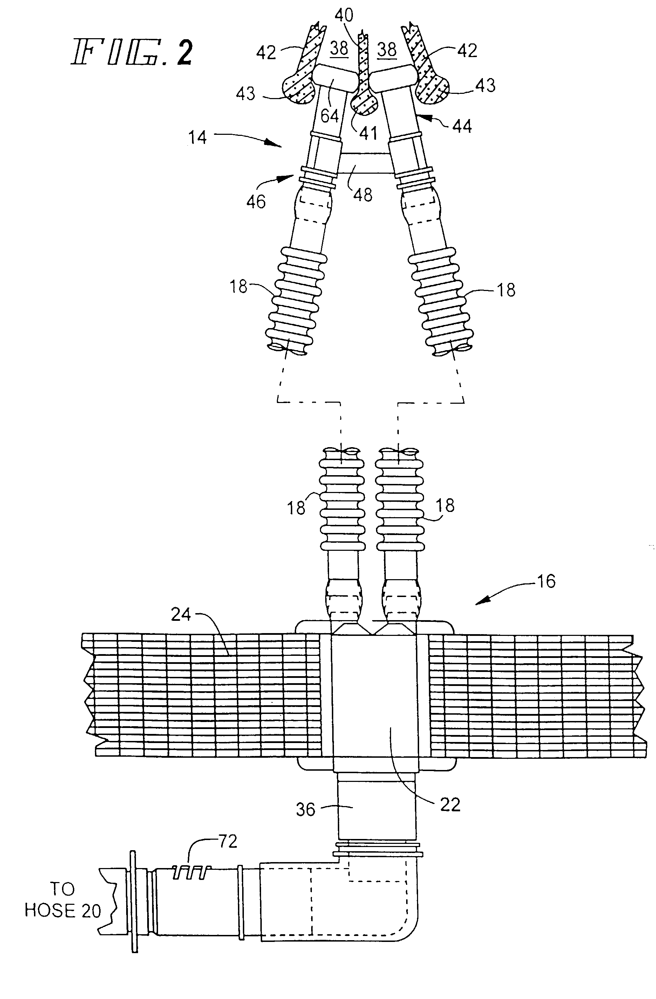

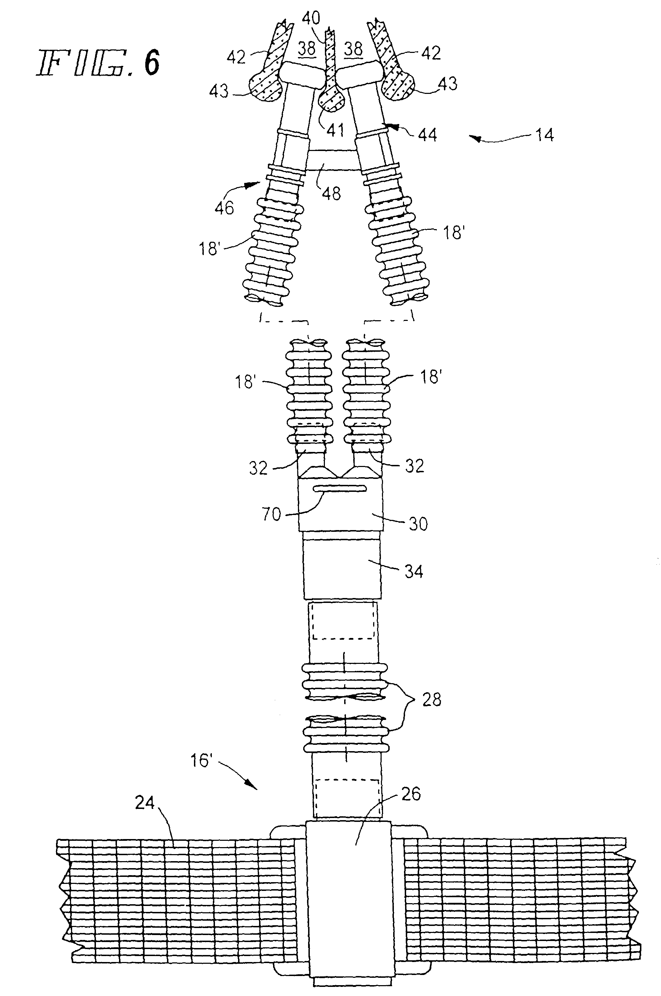

An alternative arrangement for the air supply is shown in Figure 6 wherein a straight-section coupling **26** replaces the Y-adapter **22** of Figures 1 and 2 In this alternative arrangement, the single hose topology that interconnects the air supply to the present OSA air delivery system (*i.e.* at **20** of Figure 1) is extended and maintained above the waist/chest assembly **16'**, through an upper section of 3/4" hose **28** which, in turn, connects to the lower leg of free-floating Y-adapter **30**. The two "Y" legs **32** of adapter **30** interconnect with the interface assembly **14** through twin 3/8" hoses **18'**. In this second embodiment, upper hose **28** is preferably about 6" while the smaller twin hoses **18'** are about 4" in length

Swivel joints to relieve the relative rotational, twisting motion between adjacent joined hoses, adapters and other devices may be utilized where such twisting motion might otherwise restrict the free movement between system components Such swivel joints may be of conventional design including, for example, axial joints, part no. 16906, manufactured by Sullivan. Axial joints are preferably utilized at **34** below floating Y-adapted **30**; and at **36** below the waist/chest assemblies **16,16'** of the embodiments of

### Figures 1 and 6, respectively.

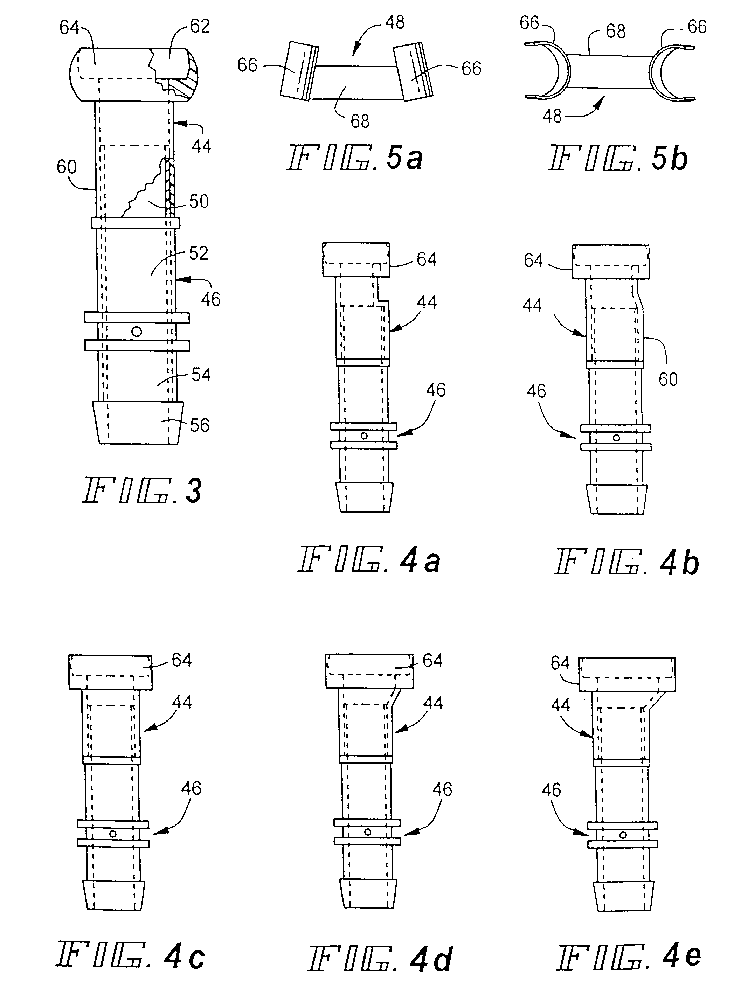

Figure 2 depicts the interface assembly **14** of the present system positioned adjacent to, and within, the nasal passages **38** of a typical patient. The patient's nose, shown in cross-section to reveal details of the nasal interface, includes septum **40** and opposed nares **42**. Interface assembly **14** serves to interconnect one or more flexible tubes **18,18'** with the nostril engagement members **44** that define the distal end of the interface assembly (see also Figures 3 and 4a-e). A pair of tubes **18,18'** is illustrated herein although it will be appreciated that a single tube and appropriate Y-adapter associated more directly with the nasal interface assembly **14** may also be alternatively employed.

In the preferred embodiment illustrated, interface assembly **14** is comprised of plural component members that facilitate the interchange of nostril engagement members **44** (as required to match the actual nose size of the particular user) and to otherwise provide greater flexibility in the design and adjustment of the nasal interface. It will be appreciated, however, that a single, *e.g.* molded plastic, interface assembly may be employed.

Referring again to Figure 2 (and Figures 3 and 4a-e), the interface assembly includes a pair of tube segments **46** held in spaced-apart relationship by bridge member **48**. As noted, a pair of nostril engagement members **44** are positioned at the distal end of each tube segment **46** to be received within, and to be retained by, the user's nostrils.

As shown in Figure 3, each tube segment **46** defines an elongate pipe for the passage of pressurized air therethrough including cylindrical top extension **50**, central barrel **52**, and hose connector **54**. Top extension **50** and hose connector **54** are adapted to frictionally receive, respectively, the engagement member **44** and flexible tube **18,18'** while the central barrel **52** is intended to accept the snap engagement of bridge member **48**. A tapered or beveled annular flange **56** may be provided at the input end of the hose connector to aid in hose retention. An aperture **58** may be provided in each tube segment **46** to permit the egress of condensation and, further, as a port through which gases may be exported during exhalation.

Still referring to Figure 3, the nostril engagement member **44** includes a generally cylindrical collar **60** adapted to be positioned, and frictionally retained, on the top extension **50** of tube segment **46** as shown. Engagement member **44** includes an annular ridge or lip **62** integrally formed around the top thereof to receive and retain an "O-ring" **64** of soft material, the latter serving as the interface and literal seal to the interior of the users nostrils. O-rings **64** are preferably fabricated of a translucent silicon material having a durometer in the order of 40.

The above-discussed bridge member **48** is best shown in Figures 5a and 5b and includes a pair of spaced-apart C-shaped collars **66** adapted, as noted, to snap-receive the central barrel region **52** of tube segment **46** therein. Collars **66** are rigidly molded to, and spaced apart, by beam **68**. As best shown in Figures 2, 5 and 6, the angle defined between the respective axes of collars **66** and therefore the corresponding angle between tube segments **46** set to approximate a typical nostril divergence, for example, about 25 degrees.

Although it is a design objective of the present invention to achieve the requisite locking engagement with the user's nose and an air-tight nostril seal through the proper selection and friction fitting of the soft O-ring material against the inner nostril walls, a secondary form of retention may be provided through a clamping force created by the angled tube segments which, in turn, bias the respective O-ring members toward one-another thereby literally grasping the comparatively hard cartilage defining septum **40**. Finally, while air supply hoses **18** and **28** are preferably flexible to facilitate the unrestrained movement of the user's head and neck, it should be noted that such hoses, notwithstanding, provide additional retention of the nasal assembly **14** by reason of the support provided by the hoses.

Each of the tube segments **46**, the bridge member **48** and engagement member **44** (except ring **64**) may preferably be fabricated of injection molded PVC or similar plastic either as separate members, as described, or as a more limited combination or single member as desired.

Figures 4a through 4e represent a family of alternative nostril engagement members **44** shown frictionally retained on the top of respective tube segments **46**. It will be appreciated that differing patient nose sizes require correspondingly different diameter O-ring seals **64**. This is particularly desirable as it is an objective of the present invention to provide an air-tight interface between the air delivery system and the nose whereby neither noise nor air leaks will be generated. It is contemplated that the present system may be delivered with a complete set of nostril engagement members **44**, *e.g.* the set shown in Figures 4a-e, permitting the user to sample and fit the system to his/her nose or, in the event a patient knows the correct engagement member size, that the system may be delivered with an appropriate single size engagement member and, further, that packages of replacement engagement members of a given single size only will be available.

It should be noted that it is not necessary that O-ring **64** be physically larger than the corresponding interior cross-section of the nostril into which the engagement member (with O-ring **64** thereon) is inserted. It has been found sufficient for some users to select an engagement member that is not over-sized, rather, that generally corresponds to the nostril cross-section and is therefore only snugly received and fitted therein. Referring to Figures 2 and 6 for illustration, it will be seen that the lower portions of the nose (and septum) define lips **41** and **43**, respectively, onto which the O-ring **64** rests and seats.

Referring again to the alternate arrangement of Figure 6, in particular, to the floating Y-adapter **30** depicted therein, it will be seen that a slot **70** is formed in the side thereof to facilitate gas communication between the pressurized air supply system, i.e. the interior of the Y-adapter, and the ambient atmospheric air surrounding patient and system alike. Slot **70** serves to bleed air from the system and, more importantly, to provide a bleed-point for carbon-dioxide and other gases exhaled during normal respiration.

In the system of Figures 1 and 2, by contrast, additional vents (other than the previously described apertures **46**) may be provided in Y-adapter **22** or, alternatively, in the supply coupling below the Y-adapter as shown at **72** in Figure 2. The substantially longer lengths of tubes **18** of Figure 2, as compared with tubes **18'** of the embodiment of Figure 6, results in a correspondingly longer vent path for (and, therefore, a greater 'volume' for the 'retention' of) waste gases. It will be appreciated, therefore, that the arrangement of Figure 6 provides improved CO₂ flushing, again, as compared with the embodiment of Figure 2 although enhanced flushing may be obtained in the system of Figure 2 by adding slots to nasal assembly **14** or by increasing the dimension of apertures **46** already provided therein.

## Claims

1. Apparatus for delivering positive pressure breathing air to a user during sleep and otherwise including air tube means for supplying a source of pressurized air, the air tube means including a first passage comprised of a single tube and a second passage comprised of twin tubes, adapter means for interconnecting first ends of each of the single tube and twin tubes in air-tight communication; nasal assembly means for attaching the second ends of the twin tubes directly to the nose of a user, without head straps and headgear, and for sealing the interface between the twin tubes and the nose, the second end of the first passage single tube adapted for connection to a source of pressurized air; stress-relieving means for securing the air means to the torso of a user whereby random forces placed on the air tube means occasioned by ordinary movement of a user during sleep are not communicated to the nasal assembly means thereby avoiding the dislodgement of such means from the nose of a user and the generation of air leaks from the interface whereby said strapless nasal assembly attachment means permits increased head and neck movement of the user and permits the user to sleep on his side and back.

2. Apparatus for delivering positive pressure breathing air to a user during sleep and otherwise including air tube means for supplying a source of pressurized air; nasal assembly means for attaching air tube means directly to the nose of a user, without head straps and headgear, and for sealing the interface between the tube means and the nose; stress-relieving means for securing the air means to the torso of a user whereby random forces placed on the air tube means occasioned by ordinary movement of a user during sleep are not communicated to the nasal assembly means thereby avoiding the dislodgement of such means from the nose of a user and the generation of air leaks from the interface whereby said strapless nasal assembly attachment means permits increased head and neck movement of the user and permits the user to sleep on his side and back.

3. The positive pressure breathing apparatus of Claim 1 in which the diameter of the twin tubes is less than the diameter of the single tube and in which the interconnecting adapter means is rigidly mounted to the stress-relieving means whereby only the lesser diameter twin tubes define the air tube means between said stress relieving means and the nasal assembly means.

4. The positive pressure breathing apparatus of Claim 1 in which the diameter of the twin tubes is less than the diameter of the single tube and including means for rigidly attaching the single tube to the stress relieving means, the single tube being attached at a point along its length intermediate the first and second ends thereof whereby the interconnecting adapter means is defined between the stress-relieving means and the nasal assembly means whereby the length of the twin tubes may be set to a predetermined short length.

5. The positive pressure breathing apparatus of Claim 1 in which the interconnecting adapter means is rigidly attached to, and forms a part of, the nasal assembly means whereby the length of the twin tubes is substantially equal to zero.

6. The positive pressure breathing apparatus of Claim 1 including vent means in the interconnecting adapter means whereby exhaled gases may be communicated to the atmosphere thereby facilitating the exchange of previously inhaled air for fresh air from the source of pressurized air.

7. The positive pressure breathing apparatus of Claim 2 in which the nasal assembly means includes first and second nostril engagement means for insertion in the nostrils of a user; the engagement means contacting the inner surface of the nostril whereby an air-tight seal between the engagement means and nostril is formed and whereby said contact defines said means for attaching the nasal assembly means without straps and other headgear.

8. The positive pressure breathing apparatus of Claim 2 in which the nasal assembly means includes first and second nostril engagement means for insertion in the nostrils of a user; the engagement means having a cross-section larger than the corresponding cross-section of the nostrils of a user at the point of contact against the inner surface of the nostril whereby the friction fit defined therebetween define an air-tight seal between the engagement means and nostril and whereby said contact defines said means for attaching the nasal assembly means without straps and other headgear.

9. The positive pressure breathing apparatus of Claim 2 in which the nasal assembly means includes first and second nostril engagement means for insertion in the nostrils of a user; the engagement means having a cross-section substantially equal to that of the corresponding cross-section of the nostrils of a user at the point of insertion and contact against the inner surface of the nostril, the engagement means further contacting the enlarged tissue defining the lower distal extension of the nose thereby defining an interfering contact between the engagement means and said enlarged tissue whereby this interfering contact defines an air-tight seal between the engagement means and nostril and whereby said contact defines said means for attaching the nasal assembly means without straps and other headgear.

10. The positive pressure breathing apparatus of Claim 2 wherein the air tube means between the stress-relieving and nasal assembly means provides support to the nasal assembly.
